# EUROPEAN PATENT APPLICATION

(11) **EP 4 613 245 A1**
(43) Date of publication of application: **10.09.2025**
(21) Application number: 25160347.8
(22) Date of filing: 26.02.2025
(51) Int. Cl.: A61F 5/445

(54) **OSTOMY BAG CHANGE DEVICE**

(30) Priority: 05.03.2024 GB 202403163
(71) Applicant: Haworth, Harvey, Longridge Preston PR3 2YP (GB)
(72) Inventor: Haworth, Harvey, Longridge Preston PR3 2YP (GB)
(74) Representative: Wilson Gunn

(57) **Abstract**

The present invention relates to a device which aids in the changing of an ostomy bag for a person, so they can change their bag anywhere, safely, quickly and hygienically.

## Description

The present invention relates to a device which aids a person in the changing of an ostomy bag.

There are many people around the world who have a stoma because of medical reasons. Common reasons include inflammatory bowel disease (Crohn's Disease or Ulcerative Colitis), diverticulitis or an obstruction to the bladder or bowel, or bowel or bladder cancer. A stoma is an opening on the surface of the abdomen which has been surgically created to divert the flow of faeces or urine and allow bodily waste to be removed from the body directly through the end of the bowel and into a collection bag. A stoma can be temporary or permanent, depending on the cause. People who have had stoma surgery are sometimes known as 'ostomates'. As used herein, the terms 'stoma 'and 'ostomy 'are used interchangeably, as they regularly are in the real world.

There are three types of stoma:
- Colostomy
- Ileostomy
- Urostomy

All three involve diversions from the bowel or bladder, though there are some differences between them.

A colostomy is the term used to describe an opening from the colon (large intestine). The surgeon will bring a part of the colon from inside the patient's body through their abdomen to the outside and stitch it down to secure it. Normally this is on the left side of the abdomen.

An ileostomy is the term used to describe an opening from the small intestine, specifically the ileum. The surgeon will bring a part of the small intestine from inside the patient's body, through their abdomen to the outside and stitch it down to secure it. Typically, this will be on the right side of the abdomen. An ileostomy is more active, with the output being looser than that of a colostomy.

A urostomy (also called an ileal conduit) is the term used to describe an opening for a person's urine. A urostomy is formed by taking a piece of a person's small intestine and attaching the ureters to it, forming a passageway for urine to pass through. One end of the tube is brought out through the abdomen to create a urostomy.

In all these cases, the ostomate does not have the ability to decide when their output (urine or faecal matter) flows into their ostomy bag, nor can they control the speed at which their output is expelled from their body into the bag. They are therefore completely at the mercy of their natural bodily functions. With an ostomy bag, there are also risks of leaks when a person is partaking in activities, or simply due to ill-fitting appliances with the bag.

However, changing an ostomy bag requires both hands, and even then, a patient still needs either to place their equipment on the ground or somehow manage to juggle all the necessary equipment in their hands while changing or emptying the bag, while ensuring the disposal bag the waste matter is being transferred into is kept open and the waste is securely transferred into it without spillage.

A stoma patient therefore needs help in changing their ostomy bag simply and quickly, and without risk of unhygienic conditions or the embarrassment of needing to ask someone for help.

Studies and the inventor's own research have shown that over 75% of stoma patients are fearful that when out and about, their ostomy bag could fill up or leak. This results in those people being fearful of doing outdoor activities, exercise, socialising, or simply leaving the house, as they do not have the facilities easily available to empty or change their ostomy bag securely and independently. Some examples of issues faced by ostomates are summarised below:

### Example 1 - Leaking bag on a walk

A stoma patient goes on a walk in the hills. At the top of a hill, they feel a leak in their bag. If they do not change it quickly, they risk faecal matter getting onto their clothes. As such, they urgently need to change their bag. Currently, they would not be able to do this without placing the necessary equipment and supplies on the ground, which is obviously unhygienic; or alternatively, asking for help from a third party, which can obviously be embarrassing, and remove a person's sense of independence.

### Example 2 - Leaking bag at work

A stoma patient works on a building site with just one portable toilet cubicle. This is limited in space and is usually not clean. Their bag has started to leak and they need to change it in a quiet place. They enter the portable toilet and must juggle all of their supplies to change their bag, risking dropping things onto unclean surfaces.

### Example 3 - Full Stoma Bag on walk

A stoma patient goes on a walk in the hills. At the top of a hill, they notice their drainable stoma bag is full. If they do not empty it, they face faecal matter getting onto their clothes due to the bag bursting or leaking. As such, they urgently need to empty their bag. Currently, their only option would be to empty it onto the ground or dig a hole and empty it into a hole. Not many people normally carry a spade or trowel around, and emptying the waste matter onto the ground can be an environmental hazard.

### Example 4 - Elderly patient with mobility issues

An elderly patient has issues with mobility. They need to empty their bag into the toilet. Currently, they face the risk of stoma output (faecal matter) splashing back up as they are unable to bend down far enough to reduce splash back.

These are all real issues which are faced by people with stomas, all of which clearly represent undesirable situations.

Other devices for changing or emptying an ostomy bag already exist, such as those described in WO2022040722. WO2022040722 describes a solid, bulky collection vessel having a hinged flap and a curved ramped portion. The ostomy bag is to be placed on the curved ramped portion for emptying into a disposal bag for the waste material to be positioned within the collection vessel.

Another device in WO2022040722 comprises a collapsible container, into which a disposal bag for the waste material is to be placed. The collapsible container is releasably attached to a slidable cassette, which can be releasably attached to a support via a drop lock. The support comprises a ramped portion having a curved ramp edge against which the user's abdomen under the ostomy pouch the support is positioned.

However, there are a number of practical disadvantages for an ostomate associated with the devices described in WO2022040722.

With regard to the solid, bulky collection vessel device, it is first of all bulkier in size, and therefore less convenient to carry around.

The curved ramped portion also presents a problem. When emptying an ostomy bag, one of an ostomate's main goals is to ensure that their stoma bag is as close to the waste receptacle (toilet, disposal bag etc) as possible. The presence of the ramp extends the distance between the ostomy bag and the waste receptacle/collection vessel, and in-fact increases the risk of the ostomate getting faecal matter onto the device or themselves either when first opening their bag, emptying the bag, or whilst cleaning the opening of it after emptying the bag.

This also holds particularly true for when an ostomate is changing a stoma bag. This is because when there is no ostomy bag over the stoma, waste output is free to drop out of the exposed stoma onto the ramped portion and down into the container. In short, the ramped portion of this collection vessel device is another piece that could get unexpected faecal output on it or cause faecal output to get onto the user's lower half (legs, feet).

This collection vessel device also does not possess any means to secure the disposal bag in an open configuration within the container, which means that there is a much greater risk of waste material not going into the disposal bag. This is critical, as disposal bags come in different thicknesses and are susceptible to collapsing in on themselves, particularly when it is being filled with something, which is not what an ostomate wants when emptying their stoma bag into it. In such a device as in WO2022040722, this would result in faecal output getting onto the outside of the disposal bag and also on the insides of the collection vessel.

This is obviously not desirable, as there is a much greater likelihood of the waste material getting on the hands of the user, and also the collection vessel will require cleaning. However, it is especially undesirable when an ostomate is out and about, as the solid, bulky collection vessel device does not have any way of fully sealing or closing it, so the vessel will smell of human faeces while the user has to carry it with them if there are no facilities to do so nearby.

Also, the curved edge on the ramped portion would not be comfortable for most people as it would not sit as comfortably against the body. It creates pressure points, particularly on the corners which risk digging into the user's skin. The curved edge would also not adapt as well to different body shapes and sizes, limiting the device's versatility and suitability for a broad range of users with varying stoma care needs.

With regard to the collapsible container device, the same disavantages described above for the ramped portion, the inability to hold a disposal bag open within the collapsable collection vessel and the curved edge on the ramped portion also apply.

In addition to the point above about the inability to secure the disposal bag in an open configuration within the container, even when within the container, without any way of securely attaching the disposal bag, a small gust of wind against the edge of a very thin plastic disposal bag could create a very different and undesirable outcome as the bag falls inwards causing output to get onto the outside of the disposal bag and the inside of the container.

In addition, the drop lock used to secure the slidable cassette to the support is not user friendly for many ostomates. Firstly, it is not apparent how it can be raised from its "lock position" when it is flush with the level of the ramped portion, without any mechanism or finger divot. This creates a difficulty for people to release the device and fold it away when they are trying to make it a quick process, particularly for those with limited dexterity which can be common among ostomates. It is seen that numerous patients who have a stoma due to Inflammatory Bowel Disease also develop arthritis as a result - otherwise known as IBD-Associated Arthritis. Additionally, the majority of ostomates at this time are older and thus naturally have a reduced dexterity.

Also, when an ostomy bag is emptied into a disposal bag in a collection vessel such as this, the bag will inevitably become heavy. This collection vessel is connected to the user only by a belt, and is not supported in any other way. Without any counterbalance to offset the increasing weight of the disposal bag in the collection vessel filling up, this means that the overall device will tip forward with the heavy full disposal bag in it, which risks some of the waste material spilling out of the bag. This collection vessel device would therefore not be steady and secure for an ostomate when in use. Moreover, the pressure exerted on the ramped feature by the increased weight of the heavy disposal bag in the collection vessel will increase the risk of failure of the device by placing strain on the attachment between the slidable cassette and support. Moreover, the pressure exerted on the ramped feature, both by the weight of the ostomy bag being placed on it, and the downward pressure exerted by the roller used to expel waste from the ostomy bag by the user into the collection vessel, will increase risk of failure of the device by placing strain on the attachment between the slidable cassette and support. Additionally, as a result of this downward pressure, the contact point between the curved edge of the ramped portion and the body of the user will also create strain resulting in the device tipping forward.

Other existing aids also suffer from similar issues.

There is therefore a great need to provide a better way for a stoma patient to be able to change or empty their ostomy bag independently, quickly, safely, easily and hygienically, where there is no fear of the disposal bag falling down or not remaining open in the process of changing or emptying the user's stoma bag, and no need for anything to be placed on the ground. The device should ideally be small, so a stoma patient can easily use it in confined spaces, such as a public or portable toilet cubicle, and also light and portable, so a stoma patient can easily carry it around when out (e.g. walking) so it can be used when a stoma bag needs to be changed or emptied.

The device of the invention would mean that a person can empty their stoma bag contents into a disposal bag and dispose of it at the next available opportunity. This would give freedom and comfort to stoma patients knowing they can change their bag anywhere themselves, safely, quickly and hygienically.

Therefore, according to a first aspect of the present invention, there is provided a device for changing or emptying an ostomy bag, the device comprising:
i) a frame to which a disposal bag can be secured in an open configuration; and
ii) a support structure to which the frame is permanently or releasably attached;
where the support structure is able to be secured to a belt; and
wherein the support structure comprises a first body section comprising an arrangement enabling it to be secured to a belt, and a second body section extending downwards from the main body section when the device is in use.

When the device is set up as described, the stoma patient can safely transfer the waste material from the ostomy bag into the disposal bag, remove the disposal bag from the frame, then close and tie up the disposal bag for the user to dispose of at the next available opportunity.

According to one embodiment of the invention, the frame has a geometrical shape, such as a square, rectangle or circle. Typically it has a plurality of sides. Typically, it has four sides, and is square or rectangular in shape, typically square. However, any suitable geometrical shape can be used if necessary.

Fundamentally, the frame is able to secure a disposal bag in an open configuration.

According to one embodiment of the invention, the frame has a plurality of locations thereon which are each able to secure a handle of the disposal bag to maintain it in the open configuration, typically under some degree of tension. This plurality of locations can comprise any elements which are able to securely hold the disposal bag in the open configuration for the waste material from the ostomy bag to be transferred into it.

As many such elements as deemed necessary may be used. Such elements may include, but are not limited to, hooks, clips, or any sort of protrusions on the frame.

The number of securing locations to secure the disposal bag may be at least 1, 2, 3, 4, 5, 6, 7, 8, 9, or 10.

The number of securing locations to secure the disposal bag may be no more than 10, 9, 8, 7, 6, 5, 4, 3, 2, 1.

The number of securing locations to secure the disposal bag may be from 1 - 10, 1 - 9, 1 - 8, 1 - 7, 1 - 6, 1 - 5, 1 - 4, 1 - 3, 1 - 2, 2 - 10, 2 - 9, 2 - 8, 2 - 7, 2 - 6, 2 - 5, 2 - 4, 2 - 3, 3 - 10, 3 - 9, 3 - 8, 3 - 7, 3 - 6, 3 - 5, 3 - 4, 4 - 10, 4 - 9, 4 - 8, 4 - 7, 4 - 6, 4 - 5, 5 - 10, 5 - 9, 5 - 8, 5 - 7, 5 - 6, 6 - 10, 6 - 9, 6 - 8, 6 - 7, 7 - 10, 7 - 9, 7 - 8, 8 - 10, 8 - 9, 9 - 10.

Preferably, the number of securing locations to secure the disposal bag is four.

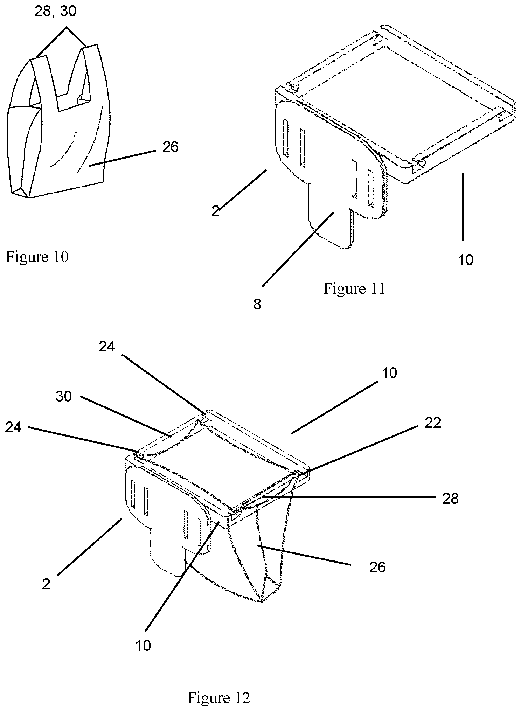

By way of non-limiting example, if the frame were a square or rectangular shape, it may have from three to six, more typically four, such locations thereon which are each able to secure a handle of the disposal bag. These securing locations are arranged on the frame in such a way as to hold the bag open under tension when the handles of the bag are engaged with them, and may be in or near each corner of the square or rectangle, so one handle of the disposal bag is secured at two adjacent securing locations, and the other handle of the disposal bag is secured at the other two adjacent securing locations (and as illustrated in Figure 12). For example, the securing locations may be on the right and left sides of the frame as viewed from the perspective of the support structure (and as illustrated in Figure 11).

In an embodiment wherein the frame is a square or rectangular shape, the securing locations may be adjacent to each other, and they may be on the left and right side of the frame as viewed from the perspective of the support structure.

In a particular preferred embodiment, each securing location may have a second securing location located directly opposite to itself on an opposite side of the frame.

In one embodiment, the securing locations may be on a top and/or bottom side of the frame as viewed from the perspective of the support structure.

In one preferred embodiment, the securing locations may be located on a corner of the sides of the frame.

In such embodiments, the securing location may be located in line with an edge of the top side and/or bottom side of the frame (as viewed from the perspective of the support structure).

In a particularly preferred embodiment, a securing location is located on each corner of the frame.

Preferably, the securing locations are positioned on each corner of the sides of the frame and are located in line with an edge of the top and/or bottom side of the frame.

Most preferably, the securing locations located at the edges of the frame closest to the user's body are located in line with an edge of the bottom side.

Beneficially, this minimises and/or mitigates the risk of stoma output (faeces) dropping through to the ground/feet due to increased disposal bag tension against the sides of the frame.

The securing locations may be positioned at least the following distances from the top or bottom frame exterior side (as viewed from the perspective of the support structure) 0 mm, 0.5 mm, 1 mm, 1.5 mm, 2 mm, 2.5 mm, 3 mm, 3.5 mm, 4 mm, 4.5 mm, 5 mm, 5.5 mm, 6 mm, 6.5 mm, 7 mm, 7.5 mm, 8 mm, 8.5 mm, 9 mm, 9.5 mm, 10 mm, 10.5 mm, 11 mm, 11.5 mm, 12 mm, 12.5 mm, 13 mm, 13.5 mm, 14 mm, 14.5 mm, 15 mm, 15.5 mm, 16 mm, 16.5 mm, 17 mm, 17.5 mm, 18 mm, 18.5 mm, 19 mm, 19.5 mm, 20 mm.

The securing locations may be positioned no more than the following distances from the top or bottom frame side (as viewed from the perspective of the support structure) 20 mm, 19.5 mm, 19 mm, 18.5 mm, 18 mm, 17.5 mm, 17 mm, 16.5 mm, 16 mm, 15.5 mm, 15 mm, 14.5 mm, 14 mm, 13.5 mm, 13 mm, 12.5 mm, 12 mm, 11.5 mm, 11 mm, 10.5 mm, 10 mm, 9.5 mm, 9 mm, 8.5 mm, 8 mm, 7.5 mm, 7 mm, 6.5 mm, 6 mm, 5.5 mm, 5 mm, 4.5 mm, 4 mm, 3.5 mm, 3 mm, 2.5 mm, 2 mm, 1.5 mm, 1 mm, 0.5 mm, 0 mm.

The securing locations may be positioned from the following distances from the top or bottom frame side (as viewed from the perspective of the support structure) 0 - 20 mm, 0 - 19.5 mm, 0 - 19 mm, 0 - 18.5 mm, 0 - 18 mm, 0 - 17.5 mm, 0 - 17 mm, 0 - 16.5 mm, 0 - 16 mm, 0 - 15.5 mm, 0 - 15 mm, 0 - 14.5 mm, 0 - 14 mm, 0 - 13.5 mm, 0 - 13 mm, 0 - 12.5 mm, 0 - 12 mm, 0 - 11.5 mm, 0 - 11 mm, 0 - 10.5 mm, 0 - 10 mm, 0 - 9.5 mm, 0-9 mm, 0 - 8.5 mm, 0 - 8 mm, 0 - 7.5 mm, 0 - 7 mm, 0 - 6.5 mm, 0 - 6 mm, 0 - 5.5 mm, 0 - 5 mm, 0 - 4.5 mm, 0 - 4 mm, 0 - 3.5 mm, 0 - 3 mm, 0 - 2.5 mm, 0 - 2 mm, 0 - 1.5 mm, 0-1 mm, 0 - 0.5 mm, 0.5 - 1 mm, 1 - 1.5 mm, 1.5 - 2 mm, 2 - 2.5 mm, 2.5 - 3 mm, 3 - 3.5 mm, 3.5 - 4 mm, 4 - 4.5 mm, 4.5 - 5 mm, 5 - 5.5 mm, 5.5 - 6 mm, 6 - 6.5 mm, 6.5 - 7 mm, 7 - 7.5 mm, 7.5 - 8 mm, 8 - 8.5 mm, 8.5 - 9 mm, 9 - 9.5 mm, 9.5 - 10 mm, 10 - 10.5 mm, 10.5 - 11 mm, 11 - 11.5 mm, 11.5 - 12 mm, 12 - 12.5 mm, 12.5 - 13 mm, 13 - 13.5 mm, 13.5 - 14 mm, 14 - 14.5 mm, 14.5 - 15 mm, 15 - 15.5 mm, 15.5 - 16 mm, 16 - 16.5 mm, 16.5 - 17 mm, 17 - 17.5 mm, 17.5 - 18 mm, 18 - 18.5 mm, 18.5 - 19 mm, 19 - 19.5 mm, 19.5 - 20 mm.

Each securing location may be positioned at the same or different distances as one another from the top or bottom frame side.

In one particularly preferred embodiment, the securing locations located at the edges closest to the user's body are positioned at a distance of from 0 - 0.5mm from the bottom frame side, most preferably substantially 0 mm from the bottom frame side.

The disposal bag may be attached to the securing locations without the need for a mechanical locking mechanism. This ensures ease of attachment and removal of the disposal bag, as well as improved hygiene, as a mechanical locking mechanism may require one or more moving parts which would require additional cleaning.

Preferably, the device does not comprise any non-disposable container.

Beneficially, this means that the device thus allows for ease of disposal of bodily fluid waste and enhanced hygiene, as there is no risk of spilling any waste into a non-disposable container.

Preferably, the frame has an open bottom.

Beneficially, this reduces the number of surfaces upon which the disposal bag may contact. This improves cleanliness and ease of maintenance of the device. Moreover, the frame having an open bottom enables the invention to work with any shape and type of disposal bag.

The frame is securely attached to the support structure. The support structure could also be characterised as a back plate. According to the invention, the frame may be securely attached to the support structure either by a permanent mode of attachment, or by a releasable mode of attachment.

Preferably, the support structure and frame may be securely and releasably connected together by any suitable means apparent to the skilled person. By releasably connected it is meant herein any mode of attachment which does not involve the use of fixing aids such as nails or screws. The frame typically has an engaging member thereon, which is able to engage releasably with a receiving member on the support structure to secure them together.

For example, they may be attached together by a simple sliding connection. In such an embodiment, the frame is secured into place by sliding the engaging member down into the receiving member. The downward pressure creates a secure connection between the engaging member and receiving member. Alternatively, the engaging member and receiving member may be a male/female engagement means, such as a plug and socket type arrangement for the secure connection.

In an alternative embodiment, the support structure and frame may be securely and permanently connected together by a non-releasable mode of attachment. By non-releasable it is meant herein any mode of attachment which involves the use of fixing aids such as nails or screws. Such a non-releasable mode of attachment may include, for example, a hinge mechanism. A hinge mechanism as used herein permanently connects the frame and support structure. For convenience storing and carrying the device, it is envisaged that the hinge allows the frame and support structure to fold up against each other.

The support structure also comprises two parts - a first body section comprising an arrangement enabling it to be secured to a belt and a second body section extending downwards from the main body section when the device is in use. The second body section typically extends downwards from the centre of the first body section, and helps to stabilise the device of the invention against the stoma patient as the waste disposal bag is filled up with waste material, by providing a counterbalance to the weight of the frame and disposal bag as it is being filled with the waste material. This second body section is typically narrower than the first body section, but does not necessarily need to be; as long as it is able to provide the necessary counterbalance to the weight of the frame and disposal bag as it is being filled with the waste material.

Preferably, the second body section may comprise rounded corners and/or edges.

Beneficially, this ensures that support structure does not cut into the body of the user during use, thereby improving comfort and the ergonomics of the device.

The second body section may have a length of at least 1 cm, 2 cm, 3 cm, 4 cm, 5 cm, 6 cm, 7 cm, 8 cm, 9 cm, 10 cm, 11 cm, 12 cm, 13 cm, 14 cm, 15 cm, 16 cm, 17 cm, 18 cm, 19 cm, 20 cm, 21 cm, 22 cm, 23 cm, 24 cm, 25 cm, 26 cm, 27 cm, 28 cm, 29 cm, 30 cm.

The second body section may have a length of no more than 30 cm, 29 cm, 28 cm, 27 cm, 26 cm, 25 cm, 24 cm, 23 cm, 22 cm, 21 cm, 20 cm, 19 cm, 18 cm, 17 cm, 16 cm, 15 cm, 14 cm, 13 cm, 12 cm, 11 cm, 10 cm, 9 cm, 8 cm, 7 cm, 6 cm, 5 cm, 4 cm, 3 cm, 2 cm, 1 cm.

The second body section may have a length from 5 - 30 cm, 5 - 25 cm, 5 - 20 cm, 5 - 15 cm, 5 - 10 cm, 10 - 30 cm, 10 - 25 cm, 10 - 20 cm, 10 - 15 cm, 15 - 30 cm, 15 - 25 cm, 15 - 20 cm, 20 - 30 cm, 20 - 25 cm, 25 - 30 cm.

The second body section may have a preferred length from 10 - 25 cm.

The second body section may have a width of at least 1 cm, 2 cm, 3 cm, 4 cm, 5 cm, 6 cm, 7 cm, 8 cm, 9 cm, 10 cm, 11 cm, 12 cm, 13 cm, 14 cm, 15 cm, 16 cm, 17 cm, 18 cm, 19 cm, 20 cm, 21 cm, 22 cm, 23 cm, 24 cm, 25 cm, 26 cm, 27 cm, 28 cm, 29 cm, 30 cm.

The second body section may have a width of no more than 30 cm, 29 cm, 28 cm, 27 cm, 26 cm, 25 cm, 24 cm, 23 cm, 22 cm, 21 cm, 20 cm, 19 cm, 18 cm, 17 cm, 16 cm, 15 cm, 14 cm, 13 cm, 12 cm, 11 cm, 10 cm, 9 cm, 8 cm, 7 cm, 6 cm, 5 cm, 4 cm, 3 cm, 2 cm, 1 cm.

The second body section may have a width from 5 - 30 cm, 5 - 25 cm, 5 - 20 cm, 5 - 15 cm, 5 - 10 cm, 10 - 30 cm, 10 - 25 cm, 10 - 20 cm, 10 - 15 cm, 15 - 30 cm, 15 - 25 cm, 15 - 20 cm, 20 - 30 cm, 20 - 25 cm, 25 - 30 cm.

The second body section may have a preferred width from 10 - 20 cm.

The second body section may have a length and width of any of the above listed sizes in combination.

In a preferred embodiment, the second body section may have a width of from 10-20 cm and a length of from 10-25 cm, in a particularly preferred embodiment, the second body section may have a width of 15 cm and a length of 11 cm.

The inventor found that the above length and width provided improved support for the frame during filling of the disposal bag.

The support structure is also typically substantially flat in shape, with no edges thereon which curve towards the ostomate when in use and provide discomfort.

As mentioned above, the first body section of the support structure comprises an arrangement enabling it to be secured to a belt. An example of a way of achieving this is that the first body section of the support structure comprises a plurality of openings thereon, through which a belt can fit. These openings are typically elongate in shape, and enable a belt of material to be threaded through them. Alternatively, a clip or similar could be used to secure the support structure to the belt. The clip may be located on the support structure or belt.

The number of openings or slots, through which a belt may be threaded may be at least 1, 2, 3, 4, 5, 6, 7, 8, 9, 10.

The number of openings or slots, through which a belt may be threaded, may be no more than 10, 9, 8, 7, 6, 5, 4, 3, 2, 1.

The number of openings or slots, through which a belt may be threaded, may be from 1 - 10, 1 - 9, 1 - 8, 1 - 7, 1 - 6, 1 - 5, 1 - 4, 1 - 3, 1 - 2, 2 - 10,2 - 9,2 - 8,2 - 7, 2 - 6, 2 - 5, 2 - 4, 2 - 3, 3 - 10, 3 - 9, 3 - 8, 3 - 7, 3 - 6, 3 - 5, 3 - 4, 4 - 10, 4 - 9, 4 - 8, 4 - 7, 4 - 6, 4 - 5, 5 - 10, 5 - 9, 5 - 8, 5 - 7, 5 - 6, 6 - 10, 6 - 9, 6 - 8, 6 - 7, 7 - 10, 7 - 9, 7 - 8, 8 - 10, 8 - 9, 9 - 10.

Preferably, the number of openings or slots, through which a belt may be threaded, is four.

The belt can be secured around the waist of the user, for example like a regular belt with a buckle, or using a material such as Velcro^{®} or similar, so the support structure is safely secured to the user. The belt is typically positioned below the ostomy bag.

The frame is then attached to the support structure, and the disposal bag is secured on the frame in an open configuration. Alternatively, the disposal bag may be secured on the frame in an open configuration, and then the frame can be attached to the support structure. The device is then ready for a person to use.

According to an embodiment of the invention, the device is designed so that the support structure is attached to the frame in a substantially perpendicular arrangement. By substantially perpendicular herein, it is meant that the angle between the support structure and the frame is between about 80-100 degrees, or 85-95 degrees.

The device of the invention may also be provided with a belt which can go through the openings in the support structure for ease of securing the device to the user. Alternatively, a person's own regular belt could be employed for this purpose.

The frame and support structure can be made from any suitable material. Non-limiting examples of materials which may be used include, for example, polypropylene, acrylonitrile butadiene styrene, polyethylene (such as high- or low-density polyethylene), a polyamide such as Nylon^{®}, polycarbonate, polymethyl methacrylate, (thermoplastic) polyurethane, silicone rubber, thermoplastic rubber, thermoplastic elastomer (TPE), polyoxymethylene, or polystyrene.

The frame and support structure can also be made by any suitable manufacturing method, such as by injection moulding.

Also provided in accordance with the present invention is a method of changing or emptying an ostomy bag, the method comprising using a device comprising:
i) a frame to which a disposal bag can be secured in an open configuration; and
ii) a support structure to which the frame is permanently or releasably attached; where the support structure is able to be secured to a belt; and
   wherein the support structure comprises a first body section comprising an arrangement enabling it to be secured to a belt, and a second body section extending downwards from the main body section when the device is in use.

Also provided in accordance with the present invention is a kit of parts, comprising:
i) a frame to which a disposal bag can be secured in an open configuration; and
ii) a support structure to which the frame is permanently or releasably attached, where the support structure is able to be secured to a belt; and wherein the support structure comprises a first body section comprising an arrangement enabling it to be secured to a belt, and a second body section extending downwards from the main body section when the device is in use.

The kit of parts may also comprise a belt to which the support structure can be secured, and/or a disposal bag.

The device of the invention may also be provided in a bespoke carry case, which is able to contain the frame and support structure as defined herein, as well as stoma supplies and one or more disposal bags. It is intended that the carry case can be affixed to the belt on the opposite side of a person's body whilst the person changes their stoma bag for easy access to supplies.

The invention will now be described further by way of example with reference to the following figures which are intended to be illustrative only and in no way limiting upon the scope of the invention.
Figure 1 depicts a front view of a support structure/back plate for the device according to the invention.
Figure 2 depicts a reverse view of the support structure/back plate for the device according to the invention.
Figure 3 depicts a perspective view of the support structure/back plate for the device according to the invention.
Figure 4 depicts a perspective view of a frame for the device according to the invention.
Figure 5 depicts a top view of a frame for the device according to the invention.
Figure 6 depicts a side view of a frame for the device according to the invention.
Figure 7 depicts a perspective view of the support structure/back plate engaging with the frame for the device according to the invention.
Figure 8 depicts an overhead view of the support structure/back plate engaging with the frame for the device according to the invention.
Figure 9 depicts an overhead view of the support structure/back plate engaged with the frame for the device according to the invention.
Figure 10 depicts a disposal bag for use with the invention.
Figure 11 depicts a perspective view of the support structure/back plate engaged with the frame for the device according to the invention.
Figure 12 depicts the present invention in its operational mode, showing a perspective view of the support structure/back plate engaged with the frame for the device according to the invention, together with a disposal bag attached to the frame.
Figure 13 depicts the present invention in its operational mode when secured to a person by a belt.
Figure 14 depicts a frame of a second embodiment of a device according to the invention.
Figure 15 depicts a top view of a frame of a second embodiment of a device according to the invention.
Figure 16 depicts a second embodiment of the present invention in its operational mode, showing a perspective view of the support structure/back plate engaged with the frame for the second embodiment of a device according to the invention, together with a disposal bag attached to the frame.

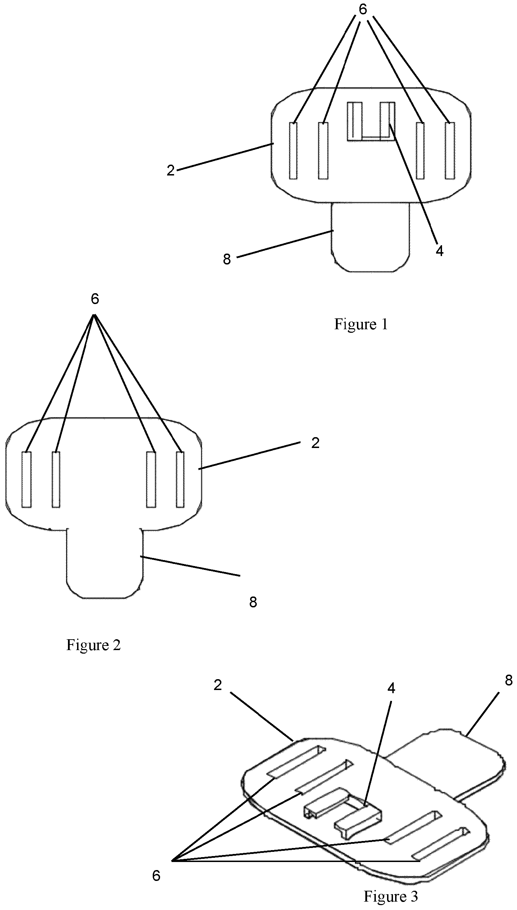

In Figure 1, a front view of a support structure/back plate 2 for a device according to the invention is shown. This embodiment depicts the alternative where the frame is releasably attached to the support structure. The support structure 2 has a receiving member 4 with which it engages with the frame (not shown in this Figure); a plurality - four in this embodiment - of openings or slots 6, through which a belt (not shown) can be threaded; and a vertical section 8 which provides the balance to the support structure 2 when the frame is engaged with the support structure 2 and the disposal bag is being filled with waste.

Figure 2 shows the reverse view of the support structure 2, with the openings 6 and the vertical section 8 visible, but obviously not the receiving member 4.

Figure 3 shows a perspective view of the support structure/back plate 2, with the receiving member 4, openings 6 and vertical section 8 all visible.

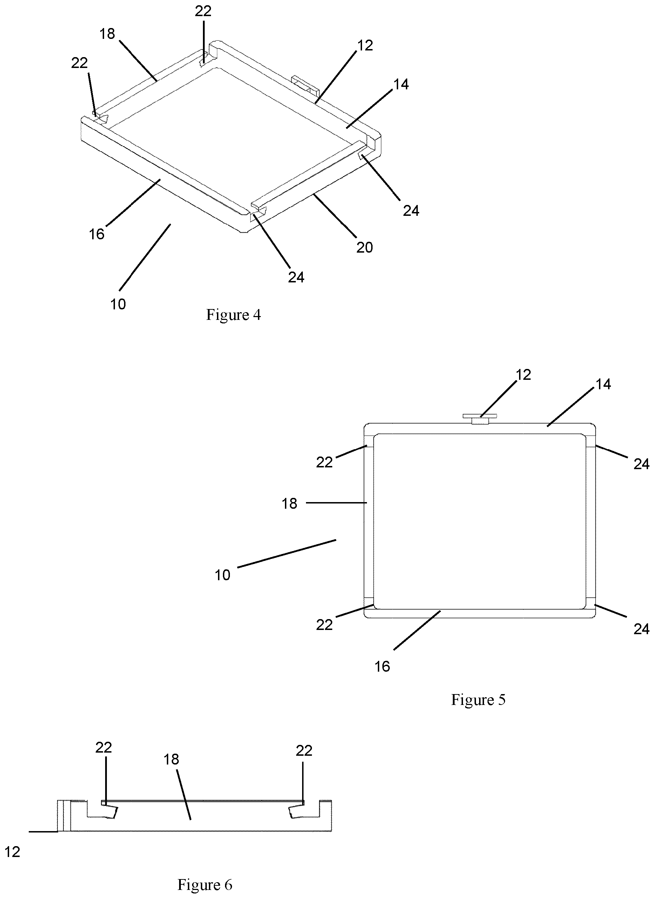

Figure 4 shows frame 10. The frame 10 has an engaging member 12 which allows the frame 10 to engage releasably with the support structure 2, as the engaging member 12 slots into the receiving member 4 of the support structure 2.

Frame 10 has four sides 14,16,18,20 in this embodiment. One side 14 has the engaging member 12 positioned thereon in the centre of the side. The opposite parallel side 16 has nothing positioned thereon, but the two perpendicular sides 18,20 connecting sides 14 and 16 each have two sets of hooking points 22,24 associated therewith. The handles of the disposal bag (not shown) are supported by these hooking points 22,24, one handle by the hooking points 22 and the other handle by the hooking points 24, to ensure the disposal bag remains in an open configuration.

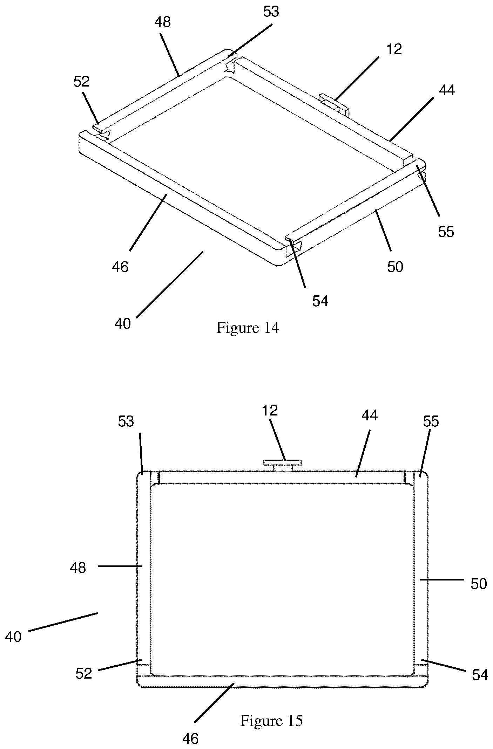

Figure 14 shows a frame 40 of a second embodiment of the invention. The frame 40 has an engaging member 42 which allows the frame 40 to engage releasably with the support structure 12, as the engaging member 42 slots into the receiving member 4 of the support structure 2.

Frame 40 has four sides 44, 46, 48, 50 in this embodiment. One side 44 has the engaging member 42 positioned thereon in the centre of the side. The opposite parallel side 46 has nothing positioned thereon, but the two perpendicular sides 48, 50 connecting sides 44 and 46 have four hooking points 52, 53, 54, 55 associated therewith. The handles of the disposal bag (not shown) are supported by these hooking points 52, 53, 54, 55, one handle by the hooking points 52, 53 and the other handle by the hooking points 54, 55 to ensure the disposal bag remains in an open configuration. The hooking points closest to the user's body 53, 55 are located at the corner of the sides of the frame 50, 48, in line with the edge of the bottom side 44.

Figure 5 shows a top view of frame 10, with the sides 14,16,18,20 being seen, and the positions of the hooking points 22,24 on the sides 18,20, and the engaging member 12 on the side 14.

Figure 15 shows a top view of a second embodiment of a frame 40, with the sides 44, 46, 48, 50 being seen, and the positions of the hooking points 52, 53, 54, 55 on the sides 48, 50, and the engaging member 12 on the side 44.

Figure 6 shows a side view of frame 10, with the hooking points 22 on the side 18, and the engaging member 12 visible on the side 14.

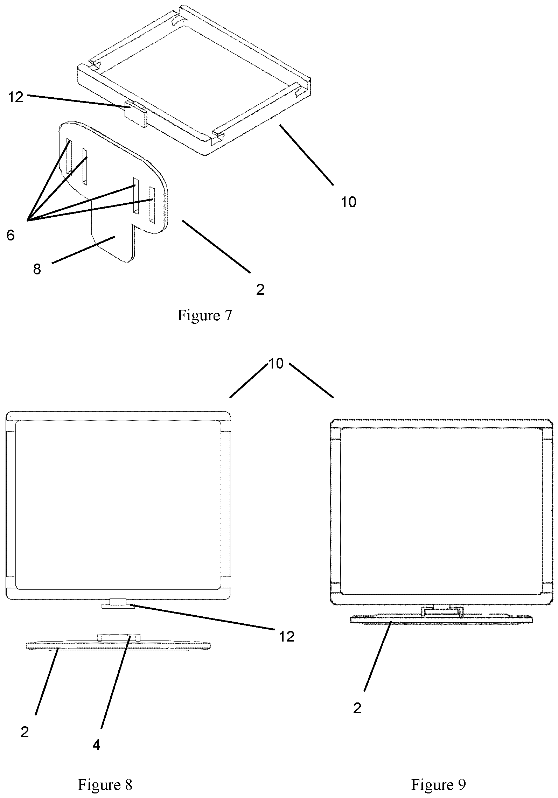

Figure 7 shows a perspective view of frame 10 in proximity to and about to engage with the support structure/back plate 2. Figure 8 shows the same arrangement, but from above.

Figure 9 then shows the overhead view of frame 10 actively engaged with the support structure 2, with the engaging member 12 having been slid into position into the receiving member 4.

Figure 10 depicts a disposal bag 26 for use with the invention, with its handles 28,30.

Figure 11 shows the perspective view of frame 10 actively engaged with the support structure 2, with the engaging member 12 (not shown in this Figure) having been slid into position into the receiving member 4 (also not shown).

Figure 12 then shows the present invention in its operational mode, with the disposal bag 26 in an open configuration by being attached to the frame 10 via its handles 28,30. Handle 28 is supported at hooking points 22, while the other handle 30 is supported at hooking points 24.

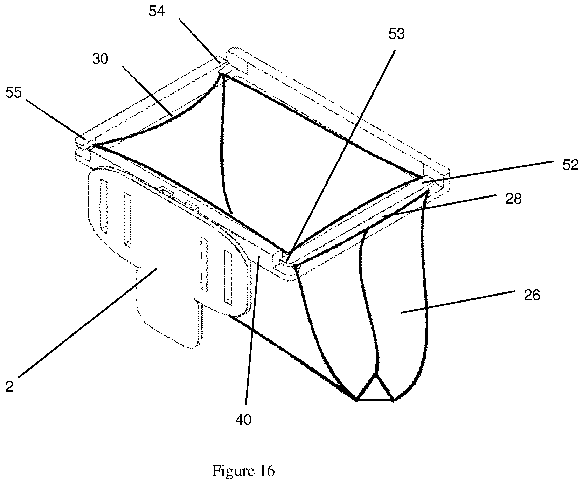

Figure 16 shows a second embodiment of the present invention in its operational mode, with the disposal bag 26 in an open configuration by being attached to the frame 40 via its handles 28, 30. Handle 28 is supported at hooking points 52, 53 while the other handle 30 is supported at hooking points 54, 55.

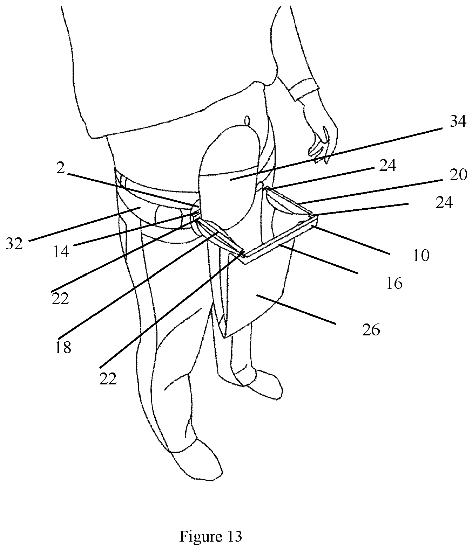

When in its operational mode, the user is able to thread a belt 32 through the openings 6 in the support structure 2 and secure the device around their waist, just below the ostomy bag 34. This is depicted in Figure 13. The support structure 2 can be seen underneath the ostomy bag 34 so that the ostomy bag 34 hangs down over or into the disposal bag 26 to ensure that all of the waste material is captured by the disposal bag 26. as with Figure 12, the disposal bag 26 is held in an open configuration by being attached to the frame 10 via its handles 28,30. Handle 28 is supported at hooking points 22, while the other handle 30 is supported at hooking points 24.

The user then has both hands free to empty or change their ostomy bag independently, quickly safely and hygienically, putting the waste material into the disposal bag 26, without making a mess.

As mentioned above, when using the device of the invention, a person may first secure the support structure 2 around their waist using the belt threaded through the openings 6. The belt may be a regular belt or may be secured using a material such as Velcro^{®} or similar. Then, either the frame 10 can be attached to the support structure 2, and the disposal bag 26 is secured on the frame 10 in an open configuration; or, alternatively, the disposal bag 26 may first be secured on the frame 10 in an open configuration, and then the frame 10 can be attached to the support structure 2. The device is then ready for a person to use.

In summary, the device of the present invention is small, light and easily portable, and can be used when:
1. a person has less space to place their stoma changing supplies;
2. an elderly person needs to empty or change their ostomy bag but they are unable to squat down to the toilet due to mobility - which puts them at risk of output (faeces) splashing back onto clothes;
3. in a work or public bathroom, when a person needs to empty or change their bag in a cubicle with limited space. Moreover, most disabled toilets also do not have stoma changing facilities; and
4. when a person is at the beach, on a mountain or even on a boat - anywhere without standard toilet/changing facilities. The device means everything can be done (changing or emptying an ostomy bag) without any assistance from a third party, and it can be done anywhere.
5. a person needs to needs to empty or change their bag quickly due to the ostomy bag being full. The speed of construction of the device can be vital as the changing and emptying of the ostomy bag can be the matter of an emergency before the bag explodes or leaks. A simpler device with fewer parts is therefore much more advantageous to a user.

There is also no requirement for a disposal container (whether collapsible or not) making the device more portable, more discreet, easier to put together and easier to use. This is due to fewer components which are also less bulky, a singular attachment means such as a sliding connection, and points to secure the disposal bag in an open configuration, which remove the need for a container.

The device of the invention is also more stable and secure due to its vertical support and counterbalance with the second body section, thus preventing the device from tipping forward when in use.

Also, the device of the invention can be used more comfortably by ostomates due to its flat back support meaning no corners dig into their body.

At the heart of the present invention is that the device gives ostomates the comfort and confidence to do more, knowing they can change their bag themselves, independently, quickly, safely and hygienically, wherever they are.

It is of course to be understood that the present invention is not intended to be restricted to the foregoing examples which are described by way of example only.

## Claims

1. A device for changing or emptying an ostomy bag, the device comprising:
i) a frame to which a disposal bag can be secured in an open configuration;
ii) a support structure to which the frame is permanently or releasably attached;
wherein the support structure is able to be secured to a belt; and
wherein the support structure comprises a first body section comprising an arrangement enabling it to be secured to a belt, and a second body section extending downwards from a main body section when the device is in use.

2. A device according to claim 1, wherein the frame has a plurality of securing locations thereon which are each able to secure a handle of the disposal bag to maintain it in the open configuration.

3. A device according to claim 2, wherein the securing locations comprise one or more selected from hooks, clips, or protrusions.

4. A device according to any preceding claim, wherein the frame has a geometrical shape.

5. A device according to any preceding claim, wherein the frame has an engaging member thereon, which is able to engage with a receiving member on the support structure.

6. A device according to claim 5, wherein the frame and support structure are attached together by a sliding connection.

7. A device according to claim 5 or claim 6, wherein the frame is secured into place by sliding the engaging member downwards into the receiving member.

8. A device according to any of claims 1-4, wherein the frame and support structure are attached together by a hinge mechanism.

9. A device according to any preceding claim, wherein the support structure is substantially flat in shape.

10. A device according to any preceding claim, wherein the wherein the support structure is secured to a belt, optionally wherein the support structure is secured to the belt via one or more openings on the support structure, or via a clip on the support structure or belt.

11. A device according to any preceding claim, wherein the second body section is narrower than the first body section.

12. A device according to any preceding claim, wherein the frame and/or support structure comprises or is made of a material selected from polypropylene, acrylonitrile butadiene styrene, polyethylene (such as high or low density polyethylene), a polyamide such as Nylon^{®}, polycarbonate, polymethyl methacrylate, (thermoplastic) polyurethane, silicone rubber, thermoplastic rubber, thermoplastic elastomer (TPE), polyoxymethylene, or polystyrene.

13. A method of changing or emptying an ostomy bag, the method comprising using a device comprising:
i) a frame to which a disposal bag can be secured in an open configuration; and
ii) a support structure to which the frame can be releasably attached;
where the support structure is able to be secured to a belt; and
wherein the support structure comprises a first body section comprising an arrangement enabling it to be secured to a belt, and a second body section extending downwards from the main body section when the device is in use.

14. A kit of parts, comprising:
i) a frame to which a disposal bag can be secured in an open configuration; and
ii) a support structure to which the frame can be releasably attached, where the support structure is able to be secured to a belt; and
wherein the support structure comprises a first body section comprising an arrangement enabling it to be secured to a belt, and a second body section extending downwards from the main body section when the device is in use.

15. A kit of parts according to claim 14, further comprising a belt and/or a disposal bag, optionally wherein the kit is provided in a carry case which is able to be affixed to the belt.
